# EUROPÄISCHE PATENTANMELDUNG

(11) **EP 2 918 308 A1**
(43) Veröffentlichungstag der Anmeldung: **16.09.2015**
(21) Anmeldenummer: 15156074.5
(22) Anmeldetag: 23.02.2015
(51) Int. Cl.: A61N 1/04

(54) **ISOLATIONSSCHLAUCH FÜR EINE ELEKTRISCHE LEITUNG ZUR MEDIZINISCHEN ANWENDUNG UND VERFAHREN ZUR HERSTELLUNG EINES SOLCHEN**

(30) Priorität: 11.03.2014 US 201461950846 P
(71) Anmelder: BIOTRONIK SE & Co. KG, 12359 Berlin (DE)
(72) Erfinder: Weitzig, Pierre, 54666 Irrel (DE); Jadwizak, Detmar, 15537 Erkner (DE); Hillebrand, Gordon, 12157 Berlin (DE); Fründt, Carsten, 13057 Berlin (DE)
(74) Vertreter: Galander, Marcus

(57) **Zusammenfassung**

Die Erfindung betrifft einen Isolationsschlauch (1) für eine elektrische Leitung zur medizinischen Anwendung, insbesondere Herzschrittmacherelektrodenleitung, Defibrillatorelektrodenleitung, elektrische Leitung zur Nervenstimulation, elektrische Leitung in Kathetern oder dergleichen, welcher, um Zugkräfte besser übertragen zu können, eine Matrix (3) bestehend aus elastischem Material und in die Matrix eingebetteten stegförmigen Elementen (5, 5a, 5c, 5e, 5f) bestehend aus steifem Kunststoffmaterial umfasst, wobei die stegförmigen Elemente (5, 5a, 5c, 5e, 5f) zumindest abschnittsweise entlang einer Schraubenlinie verlaufen, welche einen Winkel von mindestens 10° bezogen auf die Längsachse des Isolationsschlauchs ausbildet. Die Erfindung betrifft ferner ein Verfahren zur Herstellung eines derartigen Isolationsschlauchs (1) sowie eine elektrische Leitung (20) mit einem solchen Isolationsschlauch (1).

## Beschreibung

Die vorliegende Erfindung betrifft einen Isolationsschlauch für eine elektrische Leitung zur medizinischen Anwendung, insbesondere Herzschrittmacherelektrodenleitung, Defibrillatorelektrodenleitung, Elektrodenleitung zur Nervenstimulation, elektrische Leitung in Kathetern oder dergleichen, bestehend aus einem elastischen Material. Die vorliegende Erfindung betrifft ferner eine derartige elektrische Leitung sowie ein Verfahren zur Herstellung eines Isolationsschlauchs für eine derartige elektrische Leitung.

Elektronische medizinische Einrichtungen wie Herzschrittmacher (Pacemaker), Defibrillatoren oder neurologische Geräte wie Hirnschrittmacher für tiefe Hirnstimulation (Deep Brain Stimulation), Rückenmarkstimulationsgeräte, TENS (Transcutaneous Electrical Nerve Stimulator), Ablations- und Diagnostikgeräte (für Katheter) oder Geräte für muskuläre Stimulationstherapie sowie Diagnosegeräte, welche die chemischen Eigenschaften des Blutes des Patienten, anderer Körperteile oder andere Körpereigenschaften und -parameter untersuchen, verwenden häufig elektrische Leitungen, die in den Körper des Patienten geführt werden und dort zumindest über den Zeitraum der Behandlung oder Messung verbleiben. Die elektrischen Leitungen sind mit der ggf. implantierbaren elektronischen Einrichtung elektrisch leitend verbunden.

Diese medizinischen Einrichtungen umfassen üblicherweise ein ggf. körperverträgliches Gehäuse mit einer dazugehörigen elektronischen Schaltung und einer Energieversorgung, z.B. einer Batterie. Das Gehäuse besitzt mindestens eine Anschlussstelle, an der die elektrische Leitung oder die elektrische Leitungen angeschlossen werden können. Die elektrische Leitung oder die elektrische Leitungen dienen der Übertragung der elektrischen Energie oder von Signalen vom Gehäuse zu dem behandelten oder untersuchten Körperteil und umgekehrt.

Hierbei bezeichnet der Begriff "elektrische Leitung" in der Medizintechnik nicht nur ein Element, mit dem nach physikalischer Definition elektrische Energie übertragen wird, sondern umfasst eine Leitung mit einem elektrischen Leiter zusammen mit seiner umhüllenden Isolation, die häufig als Isolationsschlauch ausgebildet ist, sowie alle weiteren funktionellen Elemente, die mit der Leitung fest verbunden sind. Die elektrische Leitung umfasst beispielsweise auch die sogenannte Elektrodenspitze, mittels der die elektrische Energie in das zu behandelnde Gewebe eingeleitet wird. Häufig ist eine Elektrodenspitze auch mit Ankerelementen oder Haltestrukturen versehen, mit denen die Konstanz der räumlichen Lage der Übergangsstelle der elektrischen Energie in dem zu behandelnden Gewebe sichergestellt wird. Die Elektrodenspitze, die eine Übergangsstelle der elektrischen Energie in das Gewebe bildet, kann als Ableit-, Stimulations- oder Messelektrode ausgelegt sein.

Bei derartigen elektrischen Leitungen wird häufig zur Isolation der elektrisch leitenden Elemente außerhalb des Anschlusses und der Elektrodenspitze ein Isolationsschlauch aus Silikon verwendet. Silikone weisen eine hohe Biokompatibilität, Langzeitstabilität, eine ausreichende Härte sowie eine hohe Elastizität auf.

Aufgrund der hohen Elastizität und der geringen, üblicherweise für einen solchen Isolationsschlauch verwendeten Wandstärke kann ein Isolationsschlauch aus Silikon jedoch insbesondere im Bereich einer Ringelektrode oder der Spitze der elektrischen Leitung, welche häufig als eine Elektrodenspitze gestaltet ist, nur geringe Kräfte, insbesondere Zugkräfte, aufnehmen. Hierdurch werden die sichere Zugkraftübertragung von einer zur anderen Seite der Ringelektrode bzw. die sichere Verankerung der Elektrodenspitze erschwert.

Daher ist es wünschenswert, den Anteil der über den Isolationsschlauch übertragbaren Zugkräfte bei gleicher Wandstärke zu erhöhen, wobei die Inflatierbarkeit des Isolationsschlauchs erhalten bleiben soll, um die Montage des selben zu erleichtern.

Aus dem Dokument US 2011/0054312 A1 ist ein Katheter für medizinische Verfahren bekannt, dessen Schaft einen verstärkten Abschnitt aufweist. Zur Verstärkung können in Längsrichtung des Schafts verlaufende drahtförmige Elemente eingebracht werden. Diese Verstärkung ist für viele Anwendungen noch nicht ausreichend.

In der Druckschrift US 2012/0310180 A1 wird ein Schlauch für eine medizinische Vorrichtung beschrieben, der ein faserverstärktes Polymer enthält. Dieses Polymer ist in einer inneren Schicht des Schlauches angeordnet und bewirkt, dass dieser Schlauch nicht inflatierbar ist.

Die Aufgabe besteht somit darin, einen Isolationsschlauch sowie eine entsprechende elektrische Leitung zu schaffen, welche bei niedriger Wandstärke hohe Zugkräfte übertragen können. Hierbei soll ein derartiger Isolationsschlauch in radialer Richtung weiterhin dehnbar sein, um die Montage des Schlauches über größere Bauteile zu ermöglichen. Die Aufgabe besteht entsprechend ferner darin, ein Verfahren zur Herstellung eines derartigen Isolationsschlauchs anzugeben, welches kostengünstig und einfach durchführbar ist.

Die obige Aufgabe wird durch einen Isolationsschlauch mit den Merkmalen des Anspruchs 1, durch eine elektrische Leitung mit den Merkmalen des Anspruchs 9 sowie durch die Verfahren zur Herstellung eines Isolationsschlauchs mit den Merkmalen der Ansprüche 11 und 13 gelöst.

Insbesondere umfasst der erfindungsgemäße Isolationsschlauch eine Matrix bestehend aus elastischem Material und in die Matrix eingebetteten stegförmigen Elementen bestehend aus steifem Kunststoffmaterial, wobei die stegförmigen Elemente zumindest abschnittsweise entlang einer Schraubenlinie verlaufen, welche einen Winkel von mindestens 10° bezogen auf die Längsachse des Isolationsschlauchs ausbildet. Bevorzugt beträgt der Winkel, den die stegförmigen Elemente abschnittsweise mit der Längsachse des Isolationsschlauchs ausbilden, maximal 30°.

Der oben angegebene erfindungsgemäße Isolationsschlauch mit den entlang einer Schraubenlinie verlaufenden stegförmigen, versteifenden Elementen hat den Vorteil, dass er bei einer geringen Wandstärke hohe Zugkräfte übertragen kann, da der Isolationsschlauch durch die steifen stegförmigen Elemente in axialer Richtung, d.h. in Richtung der Längsachse, nicht dehnbar ist. Der elastische Teil des erfindungsgemäßen Isolationsschlauches lässt sich weiterhin in radialer Richtung aufweiten. Diese radiale Dehnung hat eine, wenn auch geringe, axiale Verkürzung des Isolationsschlauches zur Folge. Die schraubenförmige Verdrallung der stegförmigen Elemente erzeugt bei axialer Zugbeanspruchung eine Verankerung dieser Elemente in dem elastischen Teil des Isolationsschlauches, so dass eine Verschiebung der steifen Elemente gegenüber der elastischen Matrix verhindert wird.

Die Dehnbarkeit des Isolationsschlauches in radialer Richtung wird insbesondere auch dadurch ermöglicht, dass die stegförmigen Elemente voneinander getrennt in dem Isolationsschlauch vorliegen, insbesondere über den gesamten Umfang des Isolationsschlauches verteilt, so dass sich ein einzelnes stegförmiges Element nur über einen Teil des Umfangs erstreckt und nicht den gesamten Umfang des Isolationsschlauchs umfasst. Erfindungsgemäß lässt sich der Isolationsschlauch derart aufweiten, dass der Schlauch unter radialer Spannung über andere Bauteile gezogen werden kann, z. B. während der Montage.

Besonders einfach ist ein derartiger Isolationsschlauch aufgebaut, wenn sich mehrere stegförmige Elemente über die gesamte axiale Länge des Isolationsschlauchs erstrecken. In diesem Fall weist der erfindungsgemäße Isolationsschlauch die oben angegebenen Eigenschaften entlang seiner gesamten axialen Länge auf.

Um eine besonders gute Verankerung der stegförmigen Elemente in der Matrix aus elastischem Material zu erreichen, können in den stegförmigen Elementen Durchbrechungen vorgesehen sein, d.h. Ausnehmungen, welche sich über die gesamte Dicke des entsprechenden stegförmigen Elements erstrecken. In diese Ausnehmungen in den stegförmigen Elementen fließt die Matrix aus elastischem Material während der Herstellung eines erfindungsgemäßen Isolationsschlauchs hinein.

Ebenfalls eine gute Verankerung in der elastischen Matrix wird erreicht, wenn die stegförmigen Elemente S-förmig ausgebildet sind.

In einem bevorzugten Ausführungsbeispiel weisen die stegförmigen Elemente eine Dicke (Ausdehnung in radialer Richtung) von maximal 0,15 mm, vorzugsweise von maximal 0,1 mm, besonders bevorzugt von maximal 0,03 mm, auf. Eine solche Dicke der stegför*migen Elemente reicht aus, um die erforderliche Stabilität für den Isolationsschlauch zu bewirken.

Vorzugsweise umfassen die stegförmigen Elemente ein steifes thermoplastisches Kunststoffmaterial, vorzugsweise eine Verbindung aus der Gruppe enthaltend Polyimid, Polyurethan, Polyamid, PTFE (Polytetrafluorethylen). Ebenso ist es von Vorteil, wenn die Matrix aus einem elastischen Kunststoff besteht und vorzugsweise eine Verbindung aus der Gruppe enthaltend Silikone, Kautschuk und thermoplastische Elastomere (TPE), z.B. ein Polyetherblockamid, aufweist.

Die obige Aufgabe wird ferner gelöst durch eine elektrische Leitung mit einem oben beschriebenen Isolationsschlauch, wobei der Isolationsschlauch vorzugsweise im Bereich einer Ringelektrode und/oder einer Elektrodenspitze angeordnet ist, wobei auch eine Anordnung entlang der elektrischen Leitung auf einem großen Teil der oder ihrer gesamten Länge möglich ist. Besonders bevorzugt weist die elektrische Leitung einen inflatierbaren Ballon auf. Weiter bevorzugt ist, dass der oben beschriebene Isolationsschlauch zwischen einer innen liegenden Elektrodenzuleitung (Wendel) und der Ringelektrode und/oder der Elektrode, vorzugsweise an der Elektrodenspitze, angeordnet ist und die Elektrodenzuleitung im Bereich der jeweiligen Elektrode umgibt. Neben der jeweiligen Elektrode kann oberhalb des oben beschriebenen Isolationsschlauchs eine weitere isolierende Hülse, welche z.B. aus Silikon besteht oder den Aufbau des erfindungsgemäßen Isolationsschlauchs aufweist, vorgesehen sein.

Die erfindungsgemäße Leitung weist die oben im Zusammenhang mit dem Isolationsschlauch beschriebenen Vorteile auf. Im Falle der Anwendung eines erfindungsgemäßen Isolationsschlauchs auf einem Absatz/Ansatz einer Elektrodenspitze oder einer Ringelektrode einer elektrischen Leitung wird eine verbesserte Verankerung eines solchen verstärkten Isolationsschlauches erreicht, da nach dem herkömmlichen Weg ein sehr elastisches Material (z.B. Silikon) auf einen festen/starren Untergrund verklebt wird. Bei einer Zugbeanspruchung kommt es nach herkömmlichem Aufbau zu einem Abschälen durch Verstrecken. Wenn der erfindungsgemäße Isolationsschlauch verwendet wird, wird an dieser Stelle eine Verstreckung und demzufolge ein Abschälen der Elektrode verhindert.

Ein kostengünstiges und einfaches Herstellungsverfahren für einen solchen Isolationsschlauch weist die folgenden Schritte auf:
- Fertigung einer ersten dünnwandigen Schlauchröhre aus einem steifen KunststoffMaterial, vorzugsweise einem steifen thermoplastischen Material,
- Einbringen von Aussparungen in das steife Kunststoff-Material über einen Teil der Länge in einem mittigen Abschnitt der ersten Schlauchröhre, wobei die Aussparungen stegförmige Elemente in dem Schlauch derart definieren, dass die stegförmigen Elemente zumindest abschnittsweise entlang einer Schraubenlinie verlaufen, welche einen Winkel von mindestens 10° bezogen auf die Längsachse der Schlauchröhre ausbildet,
- Einbetten der ersten Schlauchröhre in eine Matrix bestehend aus einem elastischen Material, so dass eine zweite Schlauchröhre ausgebildet wird, welche sich aus der ersten Schlauchröhre und der umgebenden Matrix zusammensetzt,
- Abtrennen der Enden der zweiten Schlauchröhre derart, dass von der ersten Schlauchröhre lediglich zumindest ein Teil des mittigen Abschnitts stehen bleibt und den Isolationsschlauch bildet.

Besonders einfach wird das Herstellungsverfahren gestaltet, wenn zum Einbetten des ersten Schlauchs in die Matrix aus dem elastischen Material der erste Schlauch mit dem Material der Matrix umspritzt wird. Hierfür wird der erste Schlauch vorzugsweise mit einem eingelegten Kerndraht in einer Spritzgussform platziert.

Alternativ kann der erfindungsgemäße Isolationsschlauch mittels Extrusion hergestellt werden, und zwar wird aus dem elastischen Material mittels Extrusion zunächst eine Schlauchröhre ausgebildet, danach werden die stegförmigen Elemente in das extrudierte Material der Matrix, das noch nicht vollständig ausgehärtet ist, eingebettet.

Wenn erforderlich wird in einem weiteren Ausführungsbeispiel anschließend das so hergestellte Zwischenprodukt ggf. unter zusätzlicher Wärmezufuhr derart verdreht, dass die stegförmigen Elemente zumindest abschnittsweise entlang einer Schraubenlinie verlaufen, welche einen Winkel von mindestens 10° bezogen auf die Längsachse des Isolationsschlauchs ausbildet.

Weitere Ziele, Merkmale, Vorteile und Anwendungsmöglichkeiten der Erfindung ergeben sich aus der nachfolgenden Beschreibung von Ausführungsbeispielen zu der Erfindung anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

Es zeigen schematisch:
- Fig. 1: ein erstes Ausführungsbeispiel eines erfindungsgemäßen Isolationsschlauchs in einer perspektivischen Ansicht von der Seite,
- Fig. 1a: den Isolationsschlauch gemäß Fig. 1 in einer Ansicht von der Seite,
- Fig. 2: einen ersten Schritt des Herstellungsverfahrens für den Isolationsschlauch gemäß Fig. 1 in einer perspektivischen Ansicht von der Seite,
- Fig. 3: einen zweiten Schritt des Herstellungsverfahrens für den Isolationsschlauch gemäß Fig. 1 in einer perspektivischen Ansicht von der Seite,
- Fig. 4: ein zweites Ausführungsbeispiel eines erfindungsgemäßen Isolationsschlauchs in einer perspektivischen Ansicht von der Seite,
- Fig. 5: ein drittes Ausführungsbeispiel eines erfindungsgemäßen Isolationsschlauchs in einer perspektivischen Ansicht von der Seite,
- Fig. 6: ein viertes Ausführungsbeispiel eines erfindungsgemäßen Isolationsschlauchs in einer perspektivischen Ansicht von der Seite,
- Fig. 7: ein fünftes Ausführungsbeispiel eines erfindungsgemäßen Isolationsschlauchs in einer perspektivischen Ansicht von der Seite,
- Fig. 8: ein Ausführungsbeispiel für eine erfindungsgemäße elektrische Leitung in einem Querschnitt und
- Fig. 9: die elektrische Leitung gemäß Fig. 8 in einer Ansicht von der Seite.

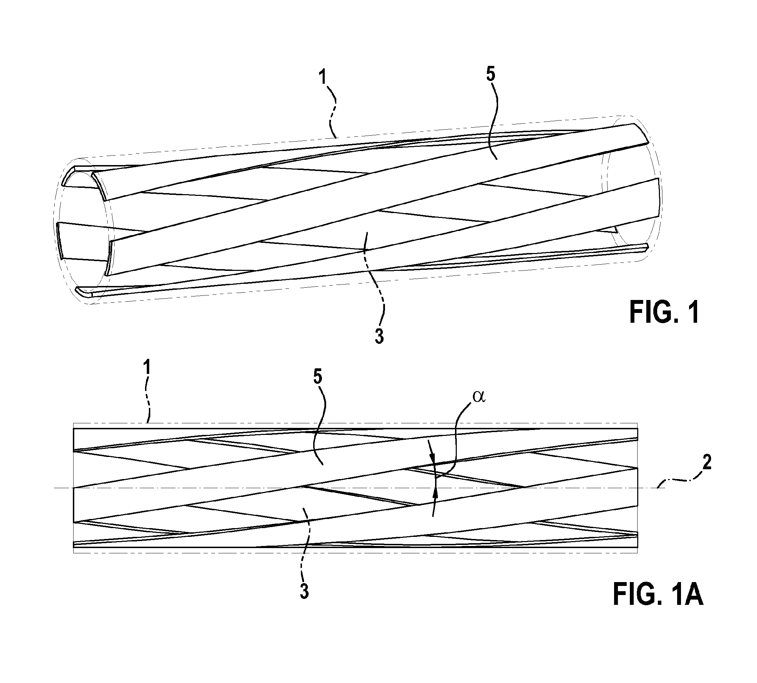

Das in Fig. 1 gestellte erste Ausführungsbeispiel eines erfindungsgemäßen Isolationsschlauchs 1 weist eine Matrix 3 z. B. aus Silikon auf, in die stegförmige Elemente 5 eingebettet sind. Die stegförmigen Elemente 5 z. B. aus Polyimid sind als streifenförmige Stege ausgebildet, welche sich über die gesamte axiale Länge des Isolationsschlauchs 1 erstrecken und um den Umfang des Isolationsschlauchs bzw. in Bezug auf die Längsachse 2 des Isolationsschlauchs schraubenförmig verlaufen. Hierbei liegen die stegförmigen Elemente 5 im Bereich der Innenwand des Isolationsschlauchs und bilden ein Teil dieser Innenwand aus. Ein derartiges stegförmiges Element 5 verläuft unter einem Winkel α von mindestens 10° zur Längsachse 2 des Isolationsschlauchs 1. Die stegförmigen Elemente sind nicht miteinander verbunden und liegen in der Matrix 3 nebeneinander über den gesamten Umfang verteilt.

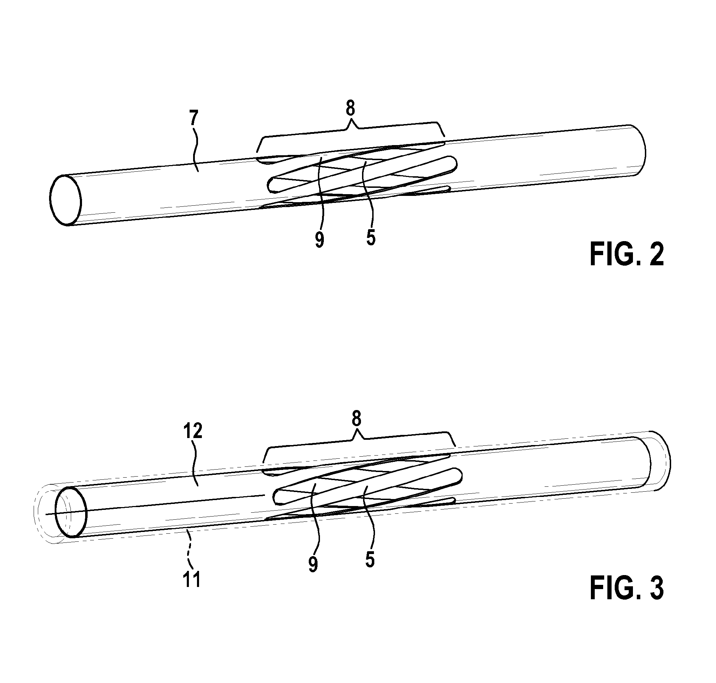

Zur Herstellung eines derartigen, in Fig. 1 dargestellten Isolationsschlauchs wird zunächst eine erste dünnwandige Schlauchröhre 7 aus einem steifen thermoplastischen Kunststoffmaterial, beispielsweise Polyimid, gefertigt, in welchen axiale, spiralförmig verlaufende Aussparungen/Schlitze 9 hineingeschnitten werden, die unter einem Winkel von mindestens 10° zur Längsachse verlaufen. Diese Schlitze 9 werden in der ersten Schlauchröhre 7 in Längsrichtung entlang eines mittigen Abschnitts 8 eingebracht, so dass die dadurch entstehenden Stege 5 durch die nicht mit Schlitzen versehenen Endabschnitte der ersten Schlauchröhre 7 in Form gehalten werden. Der Zustand nach dem Einschlitzen der ersten Schlauchröhre 7 ist in Fig. 2 gezeigt.

Diese erste Schlauchröhre 7 aus steifem Material wird nun mit einem nicht dargestellten Kerndraht in eine nicht dargestellte Spritzgussform gelegt und mit einem elastischen Material, beispielsweise Silikon, umspritzt, wie dies in Fig. 3 gezeigt ist. Das umspritzte elastische Material ist mit dem Bezugszeichen 11 und die nach der Umspritzung entstandene zweite Schlauchröhre mit dem Bezugszeichen 12 versehen. Sofern das Material der ersten Schlauchröhre 7 und des Umspritzungsmaterials 11 (hier z. B. Polyimid und Silikon) keine haftende Verbindung eingehen, sorgt diese Anordnung zumindest für einen Formschluss, der zur Übertragung von Zugkräften beiträgt. Der Zustand nach dem Umspritzen ist in Fig. 3 dargestellt.

Nach dem Umspritzen werden jeweils die Enden der zweiten Schlauchröhre 12 abgetrennt, so dass der Isolationsschlauch 1 lediglich durch den mittigen Abschnitt 8 ausgebildet wird, in welchem zuvor die Schlitze in das steife Material der ersten Schlauchröhre 7 eingebracht wurden. So entsteht der in Fig. 1 gezeigte Isolationsschlauch.

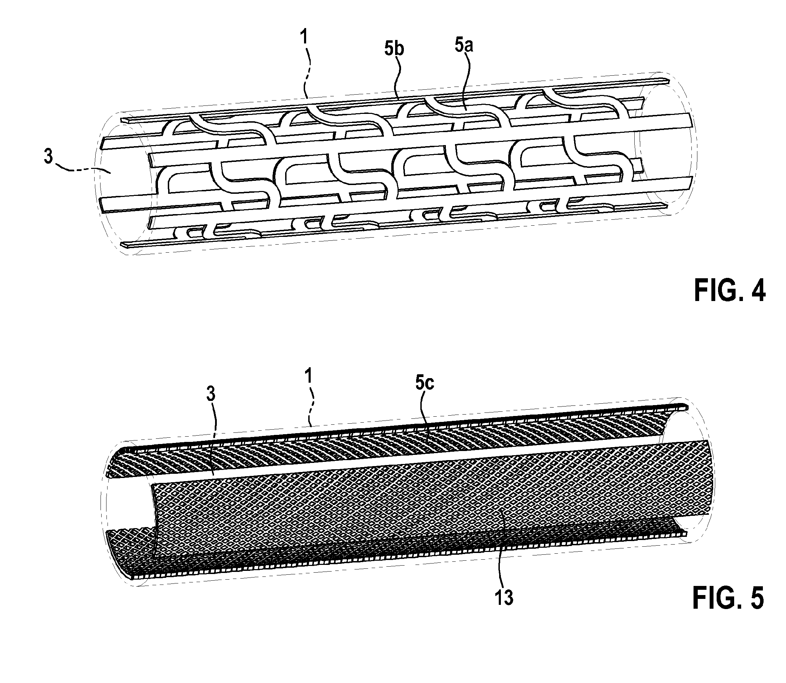

Fig. 4 zeigt ein weiteres Ausführungsbeispiel eines erfindungsgemäßen Isolationsschlauchs 1, bei dem die stegförmigen Elemente aus dem steifen Kunststoffmaterial teilweise als S-förmige Elemente 5a und in axialer Richtung verlaufende gerade Elemente 5b ausgebildet sind, welche miteinander verbunden sind. Die S-förmigen Elemente 5a verlaufen zumindest abschnittsweise entlang einer Schraubenlinie. Diese sind mit den geraden Elementen 5b verbunden.

Das in Fig. 5 dargestellte weitere Ausführungsbeispiel eines erfindungsgemäßen Isolationsschlauchs 1 weist in der elastischen Matrix 3 eingebettete stegförmige Elemente 5c auf, welche zwar jeweils insgesamt in Richtung der Längsachse 2 des Isolationsschlauchs 1 verlaufen, jedoch aus einem schräg zur Längsachse bzw. schraubenförmig verlaufenden Gitterstegen bestehen, die rhombenförmige Durchbrechungen 13 ausbilden.

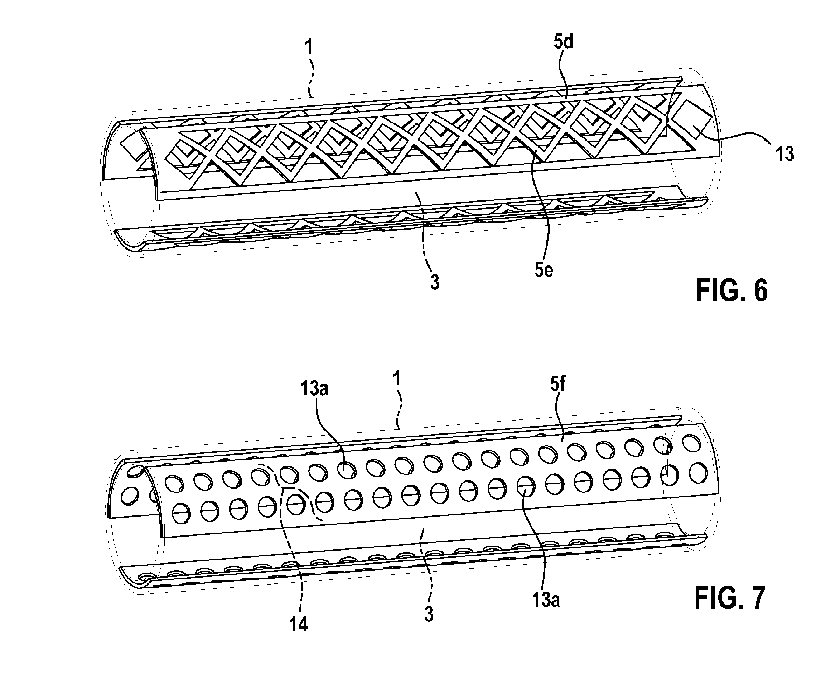

Sehr ähnlich zu dem in Fig. 5 dargestellten Ausführungsbeispiel sind die streifenförmigen Elemente des in Fig. 6 dargestellten weiteren Ausführungsbeispiels gestaltet, welche sich am Rand aus geraden, in axialer Richtung verlaufenden Elementen 5d und schräg zur Längsachse des Isolationsschlauchs 1 bzw. schraubenförmig verlaufenden, ein Gitter ausbildenden Elementen 5e zusammensetzt, wobei die geraden Elemente 5d und die schraubenförmigen Elemente 5e miteinander verbunden sind. Durch die schräg verlaufenden Elemente 5e werden ebenfalls rhombenförmige Durchbrechungen 13 realisiert.

Eine weitere Möglichkeit der Realisierung der stegförmigen Elemente ist dem in Fig. 7 dargestellten Ausführungsbeispiel zu entnehmen, wobei die stegförmigen Elemente 5f parallel zur Längsachse des Isolationsschlauchs 1 verlaufen und zwei Reihen von kreisförmigen Durchbrechungen 13a aufweisen. Hierdurch werden in den stegförmigen Elementen 5f Bereiche 14 ausgebildet, welche abschnittsweise entlang einer Schraubenlinie verlaufenden (vgl. in Fig. 7 die gestrichelte Linie mit dem Bezugszeichen 14).

Mit den in den Fig. 1 und 4 bis 7 dargestellten erfindungsgemäßen Isolationsschläuchen ist es möglich, bei einer sehr dünnwandigen Ausgestaltung hohe Zugkräfte zu übertragen ohne dabei den Vorteil einer in radialer Richtung möglichen Dehnbarkeit zu verlieren. Hierdurch ist eine Montage des Isolationsschlauchs auf größere Bauteile möglich.

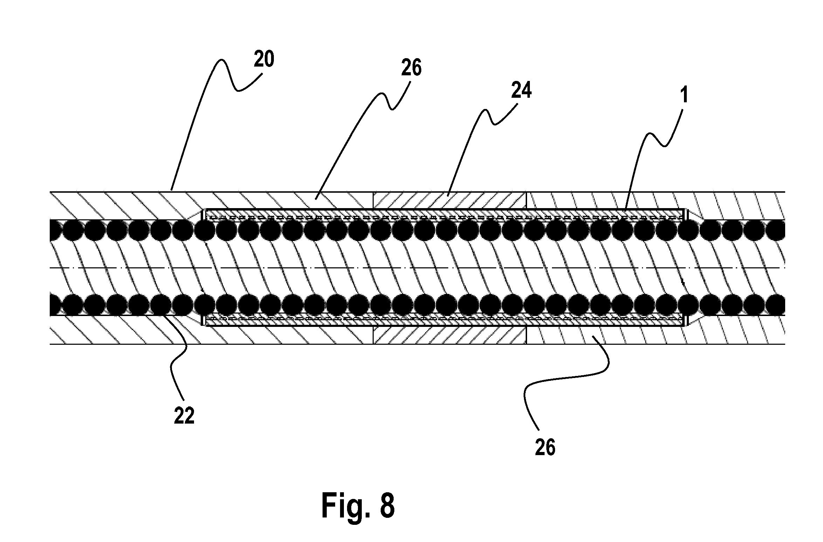

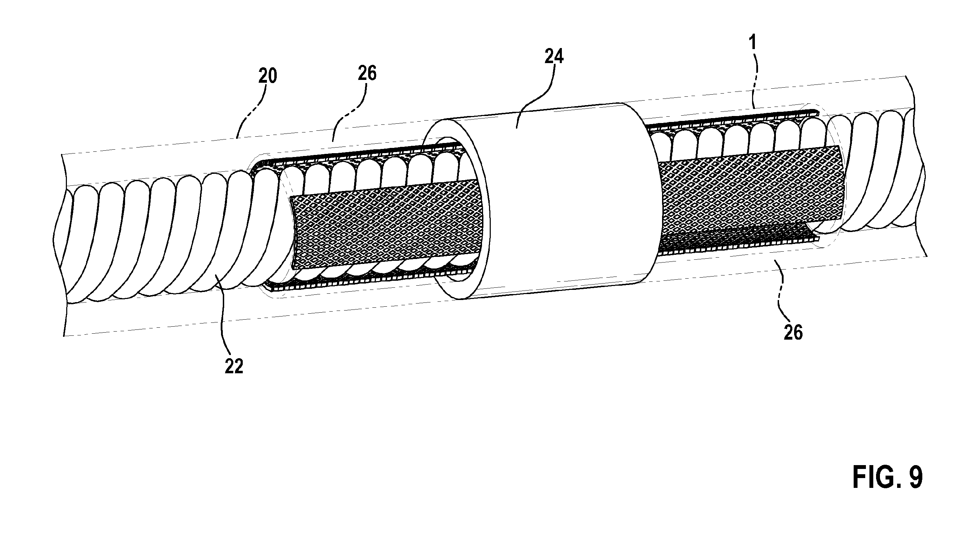

Eine erfindungsgemäße elektrische Leitung 20 ist in den Fig. 8 und 9 in Form einer Herzschrittmacherelektrode dargestellt. Diese weist eine innen liegende Elektrodenzuleitung (Wendel) 22 auf, welche die elektrische Verbindung der Elektroden zu den elektronischen Bauteilen des Herzschrittmachers schaffen. Im Bereich einer Ringelektrode 24, welche derart angeordnet ist, dass sie einen Teil der äußeren Oberfläche der elektrischen Leitung bildet, ist ein Isolationsschlauch 1 gemäß Fig. 1 vorgesehen, der zwischen der Elektrodenzuleitung 22 und der Ringelektrode 24 angeordnet ist. Die Länge des Isolationsschlauchs 1 beträgt etwa das 1,5- bis 5-fache der Länge der Ringelektrode 24, wobei die Länge von Isolationsschlauch 1 und Ringelektrode 24 jeweils in Richtung der Längsachse 2 gemessen wird. Neben der Ringelektrode 24 und oberhalb der Elektrodenzuleitung 22 bzw. des Isolationsschlauchs 1 ist eine isolierende Hülse 26 angeordnet, die als erfindungsgemäßer Isolationsschlauch ausgebildet sein kann oder lediglich aus Silikon oder einem anderen flexiblen isolierenden Material gefertigt ist.

Als Elektrodenzuleitung wird eine Coradialwendel verwendet. Alle Drähte sind einzeln isoliert und es existieren verschiedene Pole innerhalb einer Wendel. Zur Kontaktierung ist einer dieser Wendeldrähte unterbrochen (die Anzahl der zu kontaktierenden Drähte kann nach Anwendungsart und Wendeldrahtzahl variiert werden) und läuft aus dem Wendelverbund geradlinig heraus. An diesem herausstehenden Draht wird die Isolationsschicht entfernt. Über diesen Wendelbereich wird der erfindungsgemäße Isolationsschlauch 1 montiert, wo hindurch der herausstehende Wendeldraht geführt wird. Darüber wird eine geschlitzte elektrisch leitende Hülse 26 mit einer mittigen, äußeren Radialnut positioniert. Diese Hülse 26 wird durch Zusammendrücken plastisch verformt und somit auf dem Isolationsschlauch und der Wendel gegen Verrutschen fixiert. Das abisolierte Drahtende wird außen über die Ringhülse in die radiale Nut gewickelt. Das Ende des abgelegten Drahtes wird in dieser Position zur elektrischen Kontaktierung verschweißt. Anschließend wird die äußere, die eigentliche Ringhülse 24, über die Baugruppe geschoben und verschweißt.

Die in den Fig. 8 und 9 dargestellte erfindungsgemäße elektrische Leitung 20 ist deshalb von Vorteil, da, um einen elektrischen Kontakt der Elektrodenzuleitung 22 in Form einer Ringelektrode 24 nach außen zu führen, die auf der Elektrodenzuleitung 22 angeordnete isolierende Hülse 26 unterbrochen werden muss. Dieser Isolationsschlauch wird üblicherweise auf Ringabsätzen abgelegt und verklebt. Aufgrund des geringen Durchmessers und daraus resultierenden kleinen Klebeflächen, kann an dieser Stelle oft keine ausreichende Zugkraftübertragung gewährleistet werden. Der erfindungsgemäße Isolationsschlauch 1 ersetzt sonst übliche Ringabsätze und bietet dabei folgende Vorteile:
1. Die Länge kann so gewählt werden, dass eine ausreichend große Klebefläche mit der Hülse 26 bereitgestellt wird.
2. Der steife Bereich einer Ringelektrode 24 wird dadurch nicht verlängert. Das verbessert die Navigation während der Implantation der Elektrode in enge und spitzwinklige Zielvenenabgänge.
3. Durch die Verwendung von zwei gleichen Materialien, kann eine sehr gute Klebefestigkeit und damit Zugkraftübertragung und Isolation erreicht werden (z.B. besteht die Hülse 26 aus Silikon und der Isolationsschlauch 1 aus dem Matrixmaterial Silikon, die Hülse 26 und der Isolationsschlauch 1 sind verbunden mit Silikonkleber).

Daher reicht der erfindungsgemäße Isolationsschlauch 1 zwischen Elektrodenzuleitung 22 und Ringelektrode 24 hindurch und erzeugt dadurch eine Kraftübertragung von einer zur anderen Seite der Ringelektrode 24. Der erfindungsgemäße Isolationsschlauch 1 ist flexibel, braucht aber nur sehr dünnwandig zu sein. Sind der erfindungsgemäße Isolationsschlauch 1 und die Ringelektrode 24 montiert, kann auf den Enden des erfindungsgemäßen Isolationsschlauchs 1 die Hülse 26, welche entlang der gesamten elektrischen Leitung montiert wird, verklebt werden.

### Bezugszeichenliste

- 1: Isolationsschlauch
- 2: Längsachse
- 3: Matrix
- 5: stegförmiges Element
- 5a, 5b, 5c: stegförmiges Element
- 5d, 5e, 5f: stegförmiges Element
- 7: erste Schlauchröhre
- 8: mittlerer Abschnitt des ersten Schlauchs 7
- 9: Schlitz
- 11: Umspritzung
- 12: zweite Schlauchröhre
- 13, 13a: Durchbrechung
- 14: Bereich des stegförmigen Elements 5f
- 20: elektrische Leitung (Herzschrittmacherelektrode)
- 22: Elektrodenzuleitung (Wendel)
- 24: Ringelektrode
- 26: Hülse

## Patentansprüche

1. Isolationsschlauch (1) für eine elektrische Leitung zur medizinischen Anwendung, insbesondere Herzschrittmacherelektrodenleitung, Defibrillatorelektrodenleitung, elektrische Leitung zur Nervenstimulation, elektrische Leitung in Kathetern oder dergl., welcher eine Matrix (3) bestehend aus elastischem Material und in die Matrix eingebetteten stegförmigen Elementen (5, 5a, 5c, 5e, 5f) bestehend aus steifem Kunststoffmaterial umfasst, wobei die stegförmigen Elemente (5, 5a, 5c, 5e, 5f) zumindest abschnittsweise entlang einer Schraubenlinie verlaufen, welche einen Winkel von mindestens 10° bezogen auf die Längsachse des Isolationsschlauchs ausbildet.

2. Isolationsschlauch nach Anspruch 1, **dadurch gekennzeichnet, dass** die stegförmigen Elemente (5, 5a, 5c, 5e, 5f) voneinander getrennt in dem Isolationsschlauch (1) vorliegen.

3. Isolationsschlauch nach einem der vorhergehenden Ansprüche, **dadurch gekennzeichnet, dass** sich mehrere stegförmige Elemente (5, 5a, 5c, 5e, 5f) über die gesamte axiale Länge des Isolationsschlauchs (1) erstrecken.

4. Isolationsschlauch nach einem der vorhergehenden Ansprüche, **dadurch gekennzeichnet, dass** die stegförmigen Elemente (5c, 5f) Durchbrechungen (13, 13a) aufweisen.

5. Isolationsschlauch nach einem der vorhergehenden Ansprüche, **dadurch gekennzeichnet, dass** die stegförmigen Elemente (5a) S-förmig ausgebildet sind.

6. Isolationsschlauch nach einem der vorhergehenden Ansprüche, **dadurch gekennzeichnet, dass** die stegförmigen Elemente (5, 5a, 5c, 5e, 5f) eine Dicke von maximal 0,15 mm aufweisen.

7. Isolationsschlauch nach einem der vorhergehenden Ansprüche, **dadurch gekennzeichnet, dass** die stegförmigen Elemente (5, 5a, 5c, 5e, 5f) ein steifes thermoplastisches Kunststoffmaterial umfassen und vorzugsweise eine Verbindung aus der Gruppe enthaltend Polyimid, Polyurethan, Polyamid, PTFE (Polytetrafluorethylen) aufweisen.

8. Isolationsschlauch nach einem der vorhergehenden Ansprüche, **dadurch gekennzeichnet, dass** die Matrix (3) aus einem elastischen Kunststoff besteht und vorzugsweise eine Verbindung aus der Gruppe enthaltend Silikone, Kautschuk und thermoplastische Elastomere (TPE), z.B. ein Polyetherblockamid, aufweist.

9. Elektrische Leitung zur medizinischen Anwendung, insbesondere Herzschrittmacherelektrodenleitung, Defibrillatorelektrodenleitung, elektrische Leitung zur Nervenstimulation, elektrische Leitung in Kathetern oder dergl., mit einem Isolationsschlauch (1) nach einem der vorhergehenden Ansprüche, wobei der Isolationsschlauch vorzugsweise im Bereich einer Ringelektrode und/oder einer Elektrodenspitze angeordnet ist.

10. Elektrische Leitung nach Anspruch 9, **dadurch gekennzeichnet, dass** diese einen inflatierbaren Ballon aufweist.

11. Verfahren zur Herstellung eines Isolationsschlauchs (1) für eine elektrische Leitung zur medizinischen Anwendung mit den folgenden Schritten:
- Fertigung einer ersten dünnwandigen Schlauchröhre (7) aus einem steifen Kunststoff-Material, vorzugsweise aus einem steifen thermoplastischen Material,
- Einbringen von Aussparungen (9) in das steife Kunststoff-Material über einen Teil der Länge in einem mittigen Abschnitt (8) der ersten Schlauchröhre (7), wobei die Aussparungen (9) stegförmige Elemente (5) derart definieren, dass die stegförmigen Elemente (5) zumindest abschnittsweise entlang einer Schraubenlinie verlaufen, welche einen Winkel von mindestens 10° bezogen auf die Längsachse der Schlauchröhre ausbildet,
- Einbetten der ersten Schlauchröhre (7) in eine Matrix (3) bestehend aus einem elastischen Material (11), so dass eine zweite Schlauchröhre (12) ausgebildet wird, welche sich aus der ersten Schlauchröhre (7) und der umgebenden Matrix (3) zusammensetzt,
- Abtrennen der Enden der zweiten Schlauchröhre (7) derart, dass von der ersten Schlauchröhre (7) lediglich zumindest ein Teil des mittigen Abschnitts (8) stehen bleibt und den Isolationsschlauch (1) bildet.

12. Verfahren zur Herstellung nach Anspruch 11, **dadurch gekennzeichnet, dass** zum Einbetten der ersten Schlauchröhre (7) in die Matrix (3) aus dem elastischen Material (11) die erste Schlauchröhre (7) mit dem Material (11) der Matrix (3) umspritzt wird.

13. Verfahren zur Herstellung eines Isolationsschlauchs (1) für eine elektrische Leitung zur medizinischen Anwendung, wobei der Isolationsschlauch eine Matrix (3) bestehend aus elastischem Material und in die Matrix eingebettete stegförmige Elemente (5, 5a, 5c, 5e, 5f) bestehend aus steifem Kunststoffmaterial aufweist, wobei aus dem elastischen Material mittels Extrusion zunächst in eine Schlauchröhre geformt wird, danach die stegförmigen Elemente in das extrudierte Material der Matrix, das noch nicht vollständig ausgehärtet ist, eingebettet werden.

14. Verfahren nach Anspruch 13, **dadurch gekennzeichnet, dass** nach dem Einbetten der stegförmigen Elemente in das extrudierte Material der Matrix das so hergestellte Zwischenprodukt ggf. unter zusätzlicher Wärmezufuhr derart verdreht wird, dass die stegförmigen Elemente (5) zumindest abschnittsweise entlang einer Schraubenlinie verlaufen, sodass diese einen Winkel von mindestens 10° bezogen auf die Längsachse des Isolationsschlauchs ausbildet.
